# EUROPEAN PATENT SPECIFICATION

(11) **EP 2 917 356 B1**
(45) Date of publication and mention of the grant of the patent: **13.03.2019**
(21) Application number: 13853959.8
(22) Date of filing: 11.11.2013
(51) Int. Cl.: C12P 7/06, C12P 7/08, C12P 7/16, C12P 7/18, C12P 7/54

(54) **PYROLYSIS AND TORREFACTION OF BIOMASS**
PYROLYSE UND TORREFIZIERUNG VON BIOMASSE
PYROLYSE ET TORRÉFACTION DE BIOMASSE

(30) Priority: 12.11.2012 US 201261725349 P
(43) Date of publication of application: 16.09.2015
(73) Proprietor: Lanzatech New Zealand Limited, Parnell, Auckland 1052 (NZ)
(72) Inventor: SCHULTZ, Michael A, Roselle, Illinois 60172 (US); HOLMGREN, Jennifer R, Roselle, Illinois 60172 (US)
(74) Representative: J A Kemp
(86) International application number: PCT/US2013/069488
(87) International publication number: WO 2014/075013

(56) References cited:
- JP-A- 2000 152 799
- US-A1- 2003 211 585
- US-A1- 2009 239 279
- US-A1- 2012 073 199
- US-B1- 6 340 581
- GRIFFIN ET AL: "Fuel and chemical products from biomass syngas: A comparison of gas fermentation to thermochemical conversion routes", ENVIRONMENTAL PROGRESS & SUSTAINABLE ENERGY, vol. 31, July 2012 (2012-07), pages 219-224, XP002756726,
- WOLF: "Novel chemical production from gas fermentation", Platts Renewable Chemicals Conference, October 18, 2012, Houston, Texas Lanzatech presentation paper, 18 October 2012 (2012-10-18), pages 1-17, XP002756701, Retrieved from the Internet: URL:https://www.platts.com/IM.Platts.Conte nt/ProductsServices/ConferenceandEvents/20 12/xc240/presentations/Carl_Wolf.pdf [retrieved on 2016-04-19]
- SAVAGE: "The ideal biofuel", NATURE, vol. 474, 2011, pages S9-S11, XP002756702,
- DANIELL ET AL: "Commercial biomass syngas fermentation", ENERGIES, vol. 5, 19 December 2012 (2012-12-19), pages 5372-5417, XP002756727,

## Description

### FIELD

The invention provides a method for producing at least one fermentation product by microbial fermentation of a gaseous substrate produced by torrefaction or pyrolysis.

### BACKGROUND

Catalytic processes may be used to convert gases consisting primarily of CO and/or CO and hydrogen (H₂) into a variety of fuels and chemicals. Micro-organisms may also be used to convert these gases into fuels and chemicals. These biological processes, although generally slower than chemical reactions, have several advantages over catalytic processes, including higher specificity, higher yields, lower energy costs and greater resistance to poisoning.

The ability of micro-organisms to grow on CO as a sole carbon source was first discovered in 1903. This was later determined to be a property of organisms that use the acetyl coenzyme A (acetyl CoA) biochemical pathway of autotrophic growth (also known as the Woods-Ljungdahl pathway and the carbon monoxide dehydrogenase / acetyl CoA synthase (CODH/ACS) pathway). A large number of anaerobic organisms including carboxydotrophic, photosynthetic, methanogenic and acetogenic organisms have been shown to metabolize CO to various end products, namely CO₂, H₂, methane, n-butanol, acetate and ethanol. In addition to these products, the inventors have previously demonstrated that a number of other useful products carbon-based products may be obtained by fermentation using specific microorganisms or those that express particular genes.

Anaerobic bacteria, such as those from the genus Clostridium, have been demonstrated to produce ethanol from CO, CO₂ and H₂ via the acetyl CoA biochemical pathway. For example, various strains of Clostridium ljungdahlii that produce ethanol from gases are described in WO 00/68407, EP 117309, US 5,173,429, 5,593,886, and 6,368,819, WO 98/00558 and WO 02/08438. The bacterium Clostridium autoethanogenum sp is also known to produce ethanol from gases (Abrini et al., Archives of Microbiology 161, pp 345-351 (1994)).

Although processes for the fermentation of substrates containing CO and H₂ by microorganisms are known, the potential for scaling and integrating these processes into an industrial context has barely been explored. Petrochemical plants and oil refineries produce large quantities of CO as "waste" by-products. A significant proportion of the waste gases are currently sent to flare (burned), or alternatively used as a source of fuel, both of which produce the undesirable greenhouse gas CO₂. Accordingly, there exists the potential to make improvements to industrial processes by exploiting the waste gases and energy produced thereby for use in fermentation to produce desirable products while simultaneously reducing gaseous carbon emissions from industrial plants.

Liquefaction of biomass can be an economical way to obtain valuable liquid products. Biomass can be any type of woody biomass, agricultural waste, pulp and paper waste, municipal solid waste, or coal/coke. Three key processes for biomass conversion are torrefaction, pyrolysis and gasification.

Torrefaction involves subjecting biomass to relatively low temperatures (150-300 °C) in the absence of air or oxygen. Volatile materials are created and then driven off, producing a densified carbon rich solid similar to coal. The gas stream produced contains CO and CO₂.

Pyrolysis is a thermochemical decomposition of organic material at elevated temperatures (typically at temperature above 450-500 °C) without the participation of oxygen. It involves the simultaneous change of chemical composition and physical phase, and is irreversible. Pyrolysis can be characterized as 'fast' or 'slow', or somewhat in between, describing the relative time under reaction conditions. Gas, liquid, and solid products are produced, with the relative amounts depending on the temperature and reaction time. Fast pyrolysis maximizes liquid yield but is more challenging.

Gasification of biomass involves the use of oxygen/air/steam to produce syngas. Griffin et al, Environmental Progress & Sustainable Energy, vol. 31, 2012, pp 219-224 relates to fuel and chemical products from biomass syngas, in particular relating to a comparison of gas fermentation to thermochemical conversion routes.
US-A-2012/0073199 describes a method for enhancing the treatment of lignocellulose-containing materials by biotreatment, more particularly to methane production via pyrolysis.

It is an object of the invention to provide an integrated method comprising biomass liquefaction and gas fermentation to produce useful products, or at least to provide the public with a useful choice.

### SUMMARY OF INVENTION

The invention generally provides, *inter alia,* methods for the production of products by microbial fermentation of a substrate comprising CO. Thus, the invention provides a method for producing at least one product from a gaseous substrate, the method comprising:
(I)
   (a) converting at least a portion of a biomass feedstock to a gaseous substrate comprising CO by pyrolysis or torrefaction the process carried out in a liquefaction zone;
   (b) passing at least a portion of the gaseous substrate to a bioreactor comprising a culture of at least one carboxydotrophic acetogenic microorganism, and anaerobically fermenting at least a portion of the gaseous substrate to produce at least one fermentation product and a waste stream comprising a second biomass;
   (c) separating at least a portion of the second biomass from the waste stream; and
   (d) Passing a portion of the second biomass to the liquefaction zone; or
(II)
   (a) passing a biomass feedstock to a pyrolysis zone, operated at conditions to produce a gaseous substrate comprising CO, CO₂ and H₂, and at least one pyrolysis product, selected from the group consisting of pyrolysis oil and char;
   (b) passing at least a portion of the gaseous substrate to a bioreactor comprising a culture of at least one carboxydotrophic acetogenic microorganism, and anaerobically fermenting at least a portion of the gaseous substrate to produce at least one fermentation product, a waste stream comprising a second biomass and an exit gas stream comprising hydrogen;
   (c) separating at least a portion of the second biomass from the waste stream; and
   (d) passing a portion of the second biomass to the pyrolysis zone; or
(III)
   (a) passing a biomass feedstock to a torrefaction zone to produce a torrefied biomass;
   (b) passing the torrefied biomass to a pyrolysis zone to produce a gaseous substrate comprising CO, pyrolysis oil and char;
   (c) passing at least a portion of the gaseous substrate to a bioreactor comprising a culture of at least one carboxydotrophic acetogenic microorganism selected from the group consisting of Clostridium autoethanogenum, Clostridium ljundahlii, Clostridium ragsdalei and Clostridium coskatii, and anaerobically fermenting at least a portion of the gaseous substrate to produce at least one fermentation product, a waste stream comprising a second biomass and an exit gas stream comprising hydrogen;
   (d) separating at least a portion of the second biomass from the waste stream;
   (e) passing the exit gas stream to a separation zone operated at conditions to provide a hydrogen rich stream;
   (f) passing a portion of the second biomass to the torrefaction zone; and
   (g) passing the pyrolysis oil and the hydrogen rich stream to a hydrogenation zone operated at conditions to produce a hydrogenated product, said hydrogenated product having from 6 to 20 carbons.

In a first aspect, the invention provides a method of production of at least one product from a gaseous substrate, the method comprising:
(a) converting at least a portion of a biomass feedstock to a gaseous substrate comprising CO by a biomass liquefaction process selected from pyrolysis or torrefaction the process carried out in a liquefaction zone;
(b) passing at least a portion of the gaseous substrate to a bioreactor comprising a culture of at least one carboxydotrophic acetogenic microorganism, and anaerobically fermenting at least a portion of the gaseous substrate to produce at least one fermentation product and a waste stream comprising a second biomass;
(c) separating at least a portion of the second biomass from the waste stream; and
(d) Passing a portion of the second biomass to the liquefaction zone

In a particular embodiment, the substrate comprising CO further comprises CO₂ and/or H₂.

In a particular embodiment, the substrate comprising CO is synthesis gas.

In a particular embodiment the syngas is separated to provide a CO comprising substrate and a CO₂/H₂ gaseous stream. In one embodiment the CO comprising substrate is passed to a first bioreactor where it is fermented to produce one or more alcohols and/or acids and an exit gas stream comprising CO₂. In certain embodiments the exit gas stream further comprises hydrogen. In particular embodiments, the exit gas stream is combined with the CO₂/H₂ gaseous stream. In certain embodiments the combined stream is passed to a second bioreactor containing a culture of one or more micro-organisms, and fermented to produce acetate.

In a particular embodiment, the biomass liquefaction process which is pyrolysis or torrefaction is adapted so as to produce a liquefaction gas product particularly suitable for use in a gas fermentation process. For example increasing the temperature of the liquefaction process produces carbon monoxide preferentially to carbon dioxide. Fuet et al., discuss variations between gas, liquid and char production at varying temperatures (Fu et. Al., Bioresource Technology, Vol. 102, Issue 17, Sept 2011, Pg 8211-8219). In a particular embodiment, the liquefaction gas product comprises CO at a concentration of from between about 20 % to about 60%. In a particular embodiment, the liquefaction gas product comprises CO₂ at a concentration of from 0% to about 40%. In a particular embodiment, the liquefaction gas product comprises CO₂ at a concentration of from 0% to 10%. In a particular embodiment, the liquefaction gas product comprises a mixture of gases comprising CO at a concentration as described above, in combination with CO₂ at a concentration as described above and/or H₂ at a concentration as described above.

In a further particular embodiment, the liquefaction gas product comprises impurities selected from the group consisting of NH₃, NO, H₂S, HCN, SO₂ and SO₃. In a particular embodiment, at least a portion of the biomass used in the biomass liquefaction process comprises biomass recovered from the bioreactor.

In a particular embodiment, the energy produced during the liquefaction process may be used to increase the efficiency of the fermentation reaction and subsequent separation of fermentation products. In particular embodiments, the energy is used to heat or cool the fermentation substrate, or to enable separation of fermentation products, for example by distillation.

In a particular embodiment, the biomass liquefaction process comprises pyrolysis and a pyrolysis product is produced. Preferably, the pyrolysis product is pyrolysis oil, char and/or pyrolysis gas.

In a particular embodiment, at least a portion of the pyrolysis gas is passed to the bioreactor as part of the substrate comprising CO.

In a particular embodiment, the pyrolysis oil is contacted with an outlet gas stream comprising hydrogen received from the bioreactor. When the substrate comprising CO provided to the bioreactor also comprises H₂, the fermentation process fixes the CO and optionally CO₂ components of the substrate thus resulting in the outlet gas stream having a higher concentration of hydrogen. The fermentation effectively acts as a hydrogen membrane allowing H₂ to pass through unconverted and to concentrate the H₂ in the outlet gas stream compared to the substrate provided to the bioreactor. Preferably, the outlet gas stream comprising hydrogen contacts the pyrolysis oil and hydrogenates the oil to produce a hydrocarbon product having from 6 to 20 carbons. In one embodiment the hydrocarbon product is high grade kerosene suitable for use as jet fuel (JP-5, JP-8) or other processes requiring high purity kerosene. In certain embodiments, the outlet stream from the upgrading process can be used up stream as a fuel source.

In a particular embodiment, a solid and/or liquid pyrolysis product undergoes gasification and at least a portion of the gasified product is passed to the bioreactor as part of the substrate comprising CO. Preferably, the solid pyrolysis product is char and the liquid pyrolysis product is pyrolysis oil.

In a particular embodiment, the char undergoes conversion in the presence of CO₂ to form CO for addition to the substrate comprising CO. Preferably, the CO₂ for conversion is received from the bioreactor, a torrefaction process and/or a pyrolysis process.

In a particular embodiment, the biomass liquefaction process comprises torrefaction. In a particular embodiment, the torrefaction process produces one or more torrefaction gases comprising CO, CO₂ and/or H₂. Preferably, at least a portion of the one or more torrefaction gases is added to the substrate comprising CO to be passed to the fermentation process.

In a particular embodiment, the one or more biomass liquefaction processes comprises torrefaction and pyrolysis. Preferably, biomass is first subjected to torrefaction then at least a portion of at least one torrefaction product is subjected to pyrolysis after which at least a portion of at least one pyrolysis product is added to the substrate comprising CO for use in the gas fermentation.

In a particular embodiment, the substrate comprising CO further comprises CO₂ wherein the CO₂ is a product of a torrefaction, or pyrolysis process.

In a particular embodiment, the substrate comprising CO further comprises H₂ wherein the H₂ is a product of a torrefaction, or pyrolysis process.

In a particular embodiment, the one or more fermentation products are an alcohol or diol. In one embodiment the alcohol is ethanol. In an alternative embodiment the diol is 2,3-butanediol. In certain embodiments the one or more fermentation products is ethanol and 2,3-butanediol.

In a particular embodiment, the one or more fermentation products are converted to one or more alkanes.

In a particular embodiment, one or more arene compounds are obtained from the pyrolysis oil. In a further embodiment, the one or more arene compounds are combined with one or more alkanes to produce a fuel.

In a second aspect, the invention provides a method for producing at least one product from a gaseous substrate, the method comprising:
a. Passing a biomass feedstock to a pyrolysis zone, operated at conditions to produce a gaseous substrate comprising CO, CO₂ and H₂, and at least one pyrolysis product, selected from the group consisting of pyrolysis oil and char;
b. Passing at least a portion of the gaseous substrate to a bioreactor comprising a culture of at least one carboxydotrophic acetogenic microorganism, and anaerobically fermenting at least a portion of the gaseous substrate to produce at least one fermentation product, a waste stream comprising a second biomass and an exit gas stream comprising hydrogen;
c. Separating at least a portion of the second biomass from the waste stream; and
d. Passing a portion of the second biomass to the pyrolysis zone.

In one embodiment the exit gas stream is passed to a separation zone operated at conditions to provide a hydrogen rich stream. In one embodiment the pyrolysis product is pyrolysis oil, and the hydrogen rich stream and the pyrolysis oil are passed to hydrogenation zone operated at conditions to provide a hydrogenated product. In one embodiment the hydrogenated product is a hydrocarbon having between 6 and 20 carbons. In one embodiment the hydrogenation zone is operated at conditions to provide a jet fuel hydrocarbon product.

In one embodiment the gaseous substrate from the pyrolysis zone comprising CO, CO₂ and H₂, is passed to a separation zone operated at conditions to provide a CO₂ richstream and an enriched CO and H₂ gaseous substrate. In one embodiment the enriched CO and H₂ stream is passed to the bioreactor.

In one embodiment the pyrolysis product is char and the char and the CO₂ rich stream are passed to a reaction zone, operated at conditions to produce a second substrate stream comprising CO. In one embodiment, the second substrate stream comprising CO is passed to the bioreactor.

In one embodiment, the pyrolysis product is pyrolysisi oil and/or char, and the pyrolysis prodcut is passed to a gasification zone operated at conditions to produce a gasified substrate comprising CO.

In one embodiment, the biomass passed to the pyrolysis zone, is first passed to a pretreatment zone. In one embodiment the pre treatment zone is a torrefaction zone. In one embodiment the biomass is passed to the torrefaction zone, operated at coniditons to produce a terrified biomass. The terrified biomass is then passed to the pyrolysis zone.

In a third aspect, the invention provides a method for producing at least one product from a gaseous substrate, the method comprising;
a. passing a biomass feedstock to a torrefaction zone to produce a torrefied biomass;
b. passing the torrefied biomass to a pyrolysis zone to produce a gaseous substrate comprising CO, pyrolysis oil and char;
c. Passing at least a portion of the gaseous substrate to a bioreactor comprising a culture of at least one carboxydotrophic acetogenic microorganism selected from the group consisting of *Clostridium autoethanogenum*, *Clostridium ljundahlii*, *Clostridium ragsdalei* and *Clostridium coskatii*, and anaerobically fermenting at least a portion of the gaseous substrate to produce at least one fermentation product, a waste stream comprising a second biomass and an exit gas stream comprising hydrogen;
d. separating at least a portion of the second biomass from the waste stream;
e. passing the exit gas stream to a separation zone operated at conditions to provide a hydrogen rich stream;
f. passing a portion of the second biomass to the torrefaction zone; and
g. passing the pyrolysis oil and the hydrogen rich stream to a hydrogenation zone operated at conditions to produce a hydrogenated product, said hydrogenated product having from 6 to 20 carbons.

In one embodiment the torrefaction process produces a gaseous by-product stream comprising CO and at least a portion of the gaseous by-product stream is passed to the bioreactor.

In one embodiment the char is passed to a gasification zone and gasified to produce a second gaseous substrate comprising CO. In one embodiment the second gaseous substrate comprising CO is passed to the bioreactor.

In one embodiment at least two of the gas streams selected from the group consisting of the gaseous by-product stream, the second gaseous stream, or the gaseous substrate produced by the pyrolysis reaction are blended prior to being passed to the bioreactor.

The hydrogenated product is a hydrocarbon product having from 6 to 20 carbons. In one embodiment the hydrogenated product is a high grade kerosene. In one embodiment the hydrogenation zone is operated at conditions to produce a jet fuel hydrocarbon product.

Also described is a system for the production of a fermentation product comprising:
a bioreactor containing a culture of one or more micro-organisms adapted to produce the fermentation product by fermentation of a substrate comprising CO,
wherein the bioreactor is adapted to receive at least a portion of the substrate comprising CO from one or more biomass liquefaction processes.

The substrate comprising CO may further comprise CO₂ and/or H₂.

The one or more biomass liquefaction processes is selected from torrefaction and pyrolysis.
At least a portion of the biomass used in the biomass liquefaction process comprises biomass recovered from the bioreactor.

When the biomass liquefaction process comprises pyrolysis, the system further comprises a pyrolysis zone adapted to produce a pyrolysis product. Preferably, the pyrolysis product is pyrolysis oil, char and/or pyrolysis gas.

The pyrolysis zone may comprise at least one outlet adapted to pass at least a portion of a pyrolysis gas to the bioreactor.

The system may further comprise a hydrogenation zone adapted to receive:
a. pyrolysis oil from the pyrolysis reactor; and
b. an outlet gas stream comprising hydrogen from the bioreactor.

The outlet gas stream may be returned to pyrolysis zone. The outlet gas stream may beused as a fuel source.

The system may further comprise a gasification zone adapted to receive solid and/or liquid biomass and adapted to pass a gasified product to the bioreactor as part of the substrate comprising CO. Preferably, the solid and/or liquid biomass is a pyrolysis product received from the pyrolysis zone. Preferably, the solid pyrolysis product is char and the liquid pyrolysis product is pyrolysis oil.

The system may further comprise a torrefaction zone adapted to subject biomass to torrefaction to produce a terrified biomass and, an outlet adapted to pass at least a portion of one or more torrefaction gases comprising CO, CO₂ and/or H₂ to the bioreactor.

The pyrolysis zone may be adapted to receive at least a portion the torrified biomass.

The pyrolysis zone, the torrefaction zone and/or the gasification zone may further comprise one or more outlets adapted to pass at least a portion of one or more gas products to the bioreactor. Preferbly, the gas product comprises CO, CO₂ and/or H₂.

The system may comprise a char conversion zone adapted to convert char in the presence of CO₂ to form CO for addition to the substrate comprising CO. Preferably, the CO₂ for conversion is received via a gas recycling conduit from the bioreactor, the torrefaction reactor, the gasification module and/or the pyrolysis reactor.

Also described is a fermentation product when produced by the method of any of the first, second or third aspect, or the system.

The following embodiments of the method can apply to any of the aspects provided herein.

In one embodiment the carboxydotrophic acetogenic microorganism is from the genus Clostridium. In one embodiment the carboxydotrophic acetogenic microorganism is selected from the group consisting of *Clostridium autoethanogenum, Clostridium ljungdahlii*, *Clostridium ragsdalei* and *Clostridium coskatii*. In one particular embodiment, the microorganism is *Clostridium autoethanogenum* DSM23693. In another particular embodiment, the microorganism is *Clostridium ljungdahlii* DSM13528

In one embodiment, the fermentation product is an alcohol or diol. In one embodiment the alcohol is ethanol. In an alternative embodiment the diol is 2,3-butanediol. In certain embodiments the one or more fermentation products is ethanol and 2,3-butanediol. In one embodiment acetic acid is produced as a by-product of the fermentation.

In one embodiment, the method of the invention provides one or more alkanes obtained as a derivative of the one or more fermentation products. In one embodiment the one or more fermentation product is further converted to downstream products by known conversion methods, such as thermochemcical or catalytic conversion methods.

In one embodiment, the method of the invention provides one or more arene compounds obtained as a derivative of a pyrolysis oil produced according to the method of any of the above aspects. In a particular embodiment, the one or more arene compounds are combined with one or more alkanes to produce a fuel.

### BRIEF DESCRIPTION OF THE FIGURES

The accompanying figures are as follows:
Figure 1: Exemplary integrated scheme showing a system comprising biomass liquefaction processes
Figure 2: Exemplary integrated scheme showing a system and method for production of one or more products by fermentation of gaseous substrates derived from biomass liquefaction process.

### DETAILED DESCRIPTION OF THE INVENTION

### Definitions

The following is a description of the present invention, including preferred embodiments thereof, given in general terms.

As referred to herein, a "fermentation broth" is a culture medium comprising at least a nutrient media and bacterial cells.

The terms "increasing the efficiency", "increased efficiency" and the like, when used in relation to a fermentation process, include, but are not limited to, increasing one or more of the rate of growth of microorganisms catalysing the fermentation, the growth and/or product production rate at elevated product concentrations, the volume of desired product produced per volume of substrate consumed, the rate of production or level of production of the desired product, and the relative proportion of the desired product produced compared with other by-products of the fermentation.

The phrase "substrate comprising carbon monoxide" and like terms should be understood to include any substrate in which carbon monoxide is available to one or more strains of bacteria for growth and/or fermentation, for example. The substrate may be a "gaseous substrate comprising carbon monoxide" and like phrases and terms include any gas which contains a level of carbon monoxide. In certain embodiments the substrate contains at least about 20% to about 100% CO by volume, from 20% to 70% CO by volume, from 30% to 60% CO by volume, and from 40% to 55% CO by volume. In particular embodiments, the substrate comprises about 25%, or about 30%, or about 35%, or about 40%, or about 45%, or about 50% CO, or about 55% CO, or about 60% CO by volume.

While it is not necessary for the substrate to contain any hydrogen, the presence of H₂ should not be detrimental to product formation in accordance with methods of the invention. In particular embodiments, the presence of hydrogen results in an improved overall efficiency of alcohol production. For example, in particular embodiments, the substrate may comprise an approx 2:1, or 1:1, or 1:2 ratio of H₂:CO. In one embodiment the substrate comprises about 30% or less H₂ by volume, 20% or less H₂ by volume, about 15% or less H₂ by volume or about 10% or less H₂ by volume. In other embodiments, the substrate stream comprises low concentrations of H₂, for example, less than 5%, or less than 4%, or less than 3%, or less than 2%, or less than 1%, or is substantially hydrogen free. The substrate may also contain some CO₂ for example, such as about 1% to about 80% CO₂ by volume, or 1% to about 30% CO₂ by volume. In one embodiment the substrate comprises less than or equal to about 20% CO₂ by volume. In particular embodiments the substrate comprises less than or equal to about 15% CO₂ by volume, less than or equal to about 10% CO₂ by volume, less than or equal to about 5% CO₂ by volume or substantially no CO₂.

In the description which follows, embodiments of the invention are described in terms of delivering and fermenting a "gaseous substrate containing CO". In the method according to the invention, the gaseous substrate comprising CO is produced by pyrolysis or torrefication. However, it should be appreciated that the gaseous substrate may be provided in alternative forms. For example, the gaseous substrate containing CO may be provided dissolved in a liquid. Essentially, a liquid is saturated with a carbon monoxide containing gas and then that liquid is added to the bioreactor. This may be achieved using standard methodology. By way of example, a microbubble dispersion generator (Hensirisak et. al. Scale-up of microbubble dispersion generator for aerobic fermentation; Applied Biochemistry and Biotechnology Volume 101, Number 3 / October, 2002) could be used. By way of further example, the gaseous substrate containing CO may be adsorbed onto a solid support. Such alternative methods are encompassed by use of the term "substrate comprising CO" and the like.

Unless the context requires otherwise, the phrases "fermenting", "fermentation process" or "fermentation reaction" and the like, as used herein, are intended to encompass both the growth phase and product biosynthesis phase of the process. In some embodiments the bioreactor may comprise a first growth reactor and a second or further fermentation reactor. As such, the addition of metals or compositions to a fermentation reaction should be understood to include addition to any one of these reactors.

The term "bioreactor" includes a fermentation device consisting of one or more vessels and/or towers or piping arrangement, which includes the Continuous Stirred Tank Reactor (CSTR), Immobilized Cell Reactor (ICR), Trickle Bed Reactor (TBR), Bubble Column, Gas Lift Fermenter, Static Mixer, or other vessel or other device suitable for gas-liquid contact. In some embodiments the bioreactor may comprise a first growth reactor and a second or further fermentation reactor. As such, when referring to the addition of substrate to the bioreactor or fermentation reaction it should be understood to include addition to any one of these reactors where appropriate.

### Description

An integrated system comprising a biomass liquefaction process which is pyrolysis and/or torrefaction and a gas fermentation process may be used in the method of the invention to produce useful fermentation products. The biomass liquefaction processes may be adapted to produce a gaseous substrate particularly suited for use in a gas fermentation process.

The CO and CO₂ and/or H₂ is captured or channelled from the biomass liquefaction process using any convenient method. Depending on the composition of the gaseous substrate, it may also be desirable to treat it to remove any undesired impurities, before introducing it to the fermentation. For example, the substrate may be filtered or scrubbed using known methods. However, the inventors have found that the microbial culture used in the fermentation has a surprisingly high tolerance to impurities that may be found in the liquefaction products. While the liquefaction gas products may be relatively impure and appear to be unsuitable for use in a microbial fermentation the fermentation is in fact able to proceed and produce useful fermentation products.

In addition, the ultimate and relative concentration of the gases CO, CO₂ and/or H₂ may be optimised for a microbial fermentation by adjusting particular liquefaction process parameters. For example by keeping temperatures lower during the liquefaction process, liquid yields can be maximised. However by using a hotter temperature during the liquefaction process, will result in increased amount of CO. This increase in CO can be beneficial to the fermentation process, as an increase in CO gas at this stage will enable an increased ethanol yield during ther fermentation process.

### Microbial Biomass

Also described is an integrated system for the recycling of biomass from fermentation. In this, at least a portion of the biomass used in the biomass liquefaction process comprises biomass recovered from the bioreactor. The recovered biomass consists predominantly of dead cellular matter from the microorganism culture.

The biomass is removed and recycled to be processed by one or more biomass liquefaction processes, such as those described herein. It may be desirable to treat the removed biomass prior to liquefaction to remove moisture, fermentation products or to modify its characteristics in other ways.

Known liquefaction processes often use biomass comprising agricultural waste and other common biomass sources. However, these feedstocks often contain particle sizes that are too high for optimal liquefaction processing. The present invention provides a biomass feedstock recovered from a microbial fermentation. This fermentation biomass has a small particle size and is straightforward to prepare as a dry, finely divided biomass feedstock appropriate for efficient liquefaction processing.

### Torrefaction

Torrefaction is a thermo-chemical treatment of biomass in the 150 to 340 °C range in the absence of oxygen. In this process the biomass partly (especially the hemi-cellulose) decomposes, giving off various types of volatiles. The remaining torrefied biomass (solid) has approximately 30% more energy content per unit of mass. The torrefaction process produces CO, CO₂ and/or H₂ which can be used in the fermentation processes described herein. In a particular embodiment, at least a portion of one or more torrefaction gases is added to the substrate comprising CO to be passed to the fermentation process.

In a particular embodiment, the one or more biomass liquefaction processes comprises torrefaction and pyrolysis. Preferably, biomass is first subjected to torrefaction to produce torrified biomass, and then at least a portion of the torrified biomass is subjected to pyrolysis. At least one gaseous product of the pyrolysis process is passed to the bioreactor. In one embodiment, at least one gaseous product of the pyrolysis process is added to the substrate comprising CO produced by the torrefaction process. In one embodiment the at least one gaseous product of the pyrolysis process is selected from the group consisting of CO, CO₂ and H₂.

In a particular embodiment, the substrate comprising CO further comprises CO₂ wherein the CO₂ is a product of a torrefaction, or pyrolysis process.

In a particular embodiment, the substrate comprising CO further comprises H₂ wherein the H₂ is a product of a pyrolysis, or torrefaction process.

The system may further comprises a torrefaction zone adapted to subject biomass to torrefaction to produce a torrified biomass and, an outlet adapted to pass at least a portion of one or more torrefaction gases comprising CO, CO₂ and/or H₂ to the bioreactor.

In one embodiment the pyrolysis zone is adapted to receive at least a portion of the torrified biomass from the torrefaction zone.

In a particular embodiment the pyrolysis zone, the torrefaction zone and/or the gasification zone further comprise one or more outlets adapted to pass at least a portion of one or more gas products to the bioreactor. Preferbly, the gas product comprises CO, CO₂ and/or H₂.

In one embodiment, the gaseous products produced by any one of the torrefaction zone, the pyrolysis zone, or the gasification zone is passed to a separation zone operated to separate at least one portion of the gas stream(s). In one embodiment the separation zone is operated under conditions to separate CO₂ from the gas stream to produce a CO₂ rich stream, and a CO and H₂ enriched stream.

### Pyrolysis

In a particular embodiment, the biomass liquefaction process comprises pyrolysis. The pyrolysis process prodcues a gaseous substrate comprising CO and a pyrolysis product. The pyrolysis product is selected from the group consisting of pyrolysis oil and char.. Pyrolysis produces these products from biomass by heating the biomass in a low/no oxygen environment. The absence of oxygen prevents combustion. The relative yield of products from pyrolysis varies with temperature. Temperatures of 400-500 °C (752-932 °F) produce more char, while temperatures above 700 °C (1,292 °F) favor the yield of liquid and gas fuel components.

Pyrolysis occurs more quickly at the higher temperatures, typically requiring seconds instead of hours. High temperature pyrolysis is also known as gasification, and produces primarily syngas. Typical yields are 60% pyrolysis oil (also known as bio-oil), 20% biochar, and 20% syngas. By comparison, slow pyrolysis can produce substantially more char (∼50%). Once initialized, both processes produce net energy. For typical inputs, the energy required to run a "fast" pyrolyzer is approximately 15% of the energy that it outputs.

In a particular embodiment, the energy produced during the liquefaction process may be used to increase the efficiency of the fermetnation reaction and subsequent separation of fermentation products. In particular embodiments, the energy is used to heat or cool the fermentation substrate, or to enable separation of fermentation products, for example by distillation.

"Fast" pyrolysis has the advantage that it operates at atmospheric pressure and modest temperatures (400-500°C). Yields of pyrolysis oil can exceed 70%w/w. There are several kinds of fast pyrolysis reactors that may be used in the present invention. Particular embodiments comprise a reactor selected from the group consisting of a bubbling fluidized bed, a circulating fluidized beds/transport reactor, a rotating cone pyrolyzer, an ablative pyrolyzer a vacuum pyrolyzer and an Auger reactor. Fluidized bed reactors with either bubbling or circulating media are most commonly used for fast pyrolysis. Auger reactors are also used due to their simplicity and ease of control, but they do not achieve the rapid heat-rates obtained with fluidized bed reactors. Sadaka and Boateng (2009) provide a review of reactor types used for pyrolysis.

During the pyrolysis process, the organic components of biomass (i.e. cellulose, hemicellulose, and lignin) are broken down and depolymerized to form a mixture of vapours and an aersol of micron-sized droplets. Prolonging the reaction time promotes secondary reactions of the aerosols and increases the formation of low molecular weight hydrocarbons (e.g., CH₄, C₂H₆, etc.) and synthesis gas (CO and CO₂ and/or H₂). Rapid cooling and condensing of the mixture forms pyrolysis oil. In a particular embodiment, at least a portion of the pyrolysis gas is passed to the bioreactor as part of the substrate comprising CO.

Pyrolysis oil (approximately represented by C₆H₈O₄) is a complex mixture of oxygenated organic compounds (e.g., acids, alcohols, aldehydes, esters, furans, ketones, sugars, phenols and many multifunctional compounds) and water (typically around 15-30%w/w). On an elemental basis, it is compositionally similar to the parent biomass, hence it is sometimes called "liquid plant matter".

Biomass-derived pyrolysis oil is rich in carbon and can be refined in ways similar to crude petroleum. Coupled with its ease of transport and storage as compared to solid biomass material, pyrolysis oil can serve as a potential feedstock for the production of fuels and chemicals in petroleum refineries. Pyrolysis oil may be used to produce biofuels including transportation fuel. While the pyrolysis oil may be used in an unprocessed form, post-processing may be desirable to optimise pyrolysis oil for particular applications.

In a particular embodiment, the pyrolysis oil is gasified before being used in the fermentation substrate.

Pyrolysis oil contains lower quantities of trace metals and sulfur making it particularly useful as a low-emission combustion fuel. The recovery of pyrolysis oil from the pyrolysis process and separation from co-products such as char may be performed according to known methods.

The relatively high oxygen content of pyrolysis oil reduces its calorific value relative to most fossil fuels (e.g. about half that of heavy fuel oil). This high oxygen and water content can make them inferior to conventional hydrocarbon fuels in particular contexts. Additionally, phase-separation and polymerization of the liquids and corrosion of containers make storage of these liquids difficult.

Pyrolysis oil upgrading can be used to convert pyrolysis oil to gasoline by mild hydrotreating followed by hydrocracking. Such methods are well known in the art. However, hydrogen production is capital intensive and it is desirable to develop methods that increase hydrogen production and recovery efficiency, especially from low-purity streams. In the absence of hydrogen recovery, such streams end up in fuel gas or sent to flare and the high-value hydrogen component is effectively wasted.

The invention provides a method whereby an outlet gas stream comprising H₂ passes from the fermentation bioreactor to a hydrogenation zone which receives pyrolysis oil from a pyrolysis zone. The H₂ contacts the pyrolysis oil and hydrogenates the oil to produce a hydrocarbon product. The hydrocarbon product having between 6 and 20 carbons. In one embodiment the hydrocarbon product is high grade kerosene. In one embodiment the hydrogenation zone is operated unders conditions to produce a jet fuel hydrocarbon product. In one embodiment, the hydrogenation occurs in a steam reformer module.

In a particular embodiment, the pyrolysis oil is contacted with an outlet gas stream comprising hydrogen received from the bioreactor. A substrate comprising CO is provided to the bioreactor. The substrate comprises gases that may have been produced as by-products of the pyrolysis process, or an alternative biomass liquefaction process. In a particular embodiment, the substrate comprising CO also comprises H₂. The fermentation process fixes at least a portion of the CO and optionally CO₂ components of the substrate thus resulting in the outlet gas stream having a higher concentration of hydrogen.

The present invention provides a method of using the fermentation reaction as a hydrogen purification apparatus then using the hydrogen to upgrade the pyrolysis oil to superior quality biofuels. These high quality end-products can be produced without the need for costly storage or transport of the pyrolysis oil and without the requirement for high purity hydrogen to be obtained and stored for use in the pyrolysis oil upgrading process. The fermentation effectively acts as a hydrogen membrane allowing H₂ to pass through unconverted and to concentrate the H₂ in the outlet gas stream compared to the substrate provided to the bioreactor.

Where the output stream comprises H₂ and unacceptable levels of impurities or other gas species, further purification may be desirable before pyrolysis oil upgrading. Methods of purification will be known to those of skill in the art, and may include the use of a pressure swing adsorption process. A Pressure Swing Adsorption (PSA) process may be used to recover hydrogen from an impure stream or to increase the purity of hydrogen in the stream. The gas stream comprising H₂ enters a molecular sieve system which adsorbs CO₂, CO, CH₄ N₂ and H₂O at high pressure. Hydrogen is able to pass through the sieve and is collected at approximately 65-90% yield (higher yield being associated with lower final H₂ product purity). Once saturated, the sieve is depressurised then the desorbed gases are swept out using the smallest possible quantity of hydrogen product. The extent of regeneration is a function of pressure, as a greater quantity of adsorbed species is released at lower regeneration pressures. This, in turn, leads to greater hydrogen recovery. Therefore, regeneration pressures of close to atmospheric pressure maximize hydrogen recovery. The vessel is then repressurised with hydrogen ready for the next period as adsorber. Commercial systems will typically have three or four vessels to give a smooth operation. A typical gas stream output from the PSA step would include the following: H₂ (approximately 7-27%), CO₂, CO and CH₄.

### Gasification (the method of the invention requires pyrolysis or torrefaction)

In a particular disclosure, a solid and/or liquid feedstock undergoes gasification in a gasification zone adapted to receive solid and/or liquid biomass. At least a portion of the gasified product is passed to the bioreactor as part of the substrate comprising CO. In a particular embodiment, the feedstock is a solid pyrolysis product, for example char, or a liquid pyrolysis product, for example pyrolysis oil.

During gasification, the feedstock undergoes the following processes:
a. Dehydration. Occurs at approximately 100°C. Typically the resulting steam is mixed into the gas flow and may be involved with subsequent chemical reactions, notably the water-gas reaction if the temperature is sufficiently high enough (see step 5);
b. pyrolysis process occurs at around 200-300°C. Volatiles are released and char is produced, resulting in up to 70% weight loss for coal. The process is dependent on the properties of the carbonaceous material and determines the structure and composition of the char, which will then undergo gasification reactions.
c. combustion process occurs as the volatile products and some of the char reacts with oxygen to primarily form carbon dioxide and small amounts of carbon monoxide, which provides heat for the subsequent gasification reactions;
d. gasification occurs as the char reacts with carbon, steam and CO₂ to produce carbon monoxide and hydrogen. In addition, the reversible gas phase water gas shift reaction reaches equilibrium very fast at the temperatures in a gasifier. This balances the concentrations of carbon monoxide, steam, carbon dioxide and hydrogen.

### Char conversion

Char is a solid charcoal produced by pyrolysis of biomass. Char may be referred to as bio-char when it is used for particular purposes, such as a soil amendment to increase soil fertility, raise agricultural productivity or to improve low grade soils. The use of char can reduce deforestation and has been postulated as a method of mitigating global warming by carbon sequestration.

The quality of char varies depending on the source and production process. When used as a soil amendment, char can improve water quality, reduce soil emissions of greenhouse gases, reduce nutrient leaching, reduce soil acidity, and reduce irrigation and fertilizer requirements.

Also provided is a fermentation process comprising the use of a substrate comprising CO wherein at least a portion of the substrate is produced by a char conversion process.

The char undergoes conversion in a char conversion module in the presence of CO₂ to form CO. Preferably, the CO₂ for conversion is received in the char conversion module via a gas recycling conduit from the bioreactor, the torrefaction zone, and/or the pyrolysis zone.

### Products

The method of the invention provides fermentation products. In a particular embodiment, the fermentation product is an alcohol or diol. In one embodiment the alcohol is ethanol. In an alternative embodiment the diol is 2,3-butanediol. In certain embodiments the one or more fermentation products is ethanol and 2,3-butanediol. Downstream processing of the fermentation products may produce derivatives such as alkanes or other hydrocarbons.

The method of the invention also provides one or more arene compounds that may be obtained by processing of the pyrolysis oil according to the methods and systems described herein. In particular embodiments, the one or more arene compounds may be combined with alkanes to produce other fuels and compounds, particularly transportation fuels.

It will be appreciated that for growth of the bacteria and the production of products to occur, in addition to the CO-containing substrate gas, a suitable liquid nutrient medium will need to be fed to the bioreactor.

In particular embodiments, the fermentation occurs in an aqueous culture medium. In particular embodiments, the fermentation of the substrate takes place in a bioreactor.

The substrate and media may be fed to the bioreactor in a continuous, batch or batch fed fashion. A nutrient medium will contain vitamins and minerals sufficient to permit growth of the micro-organism used. Anaerobic media suitable for fermentation using CO are known in the art. For example, suitable media are described Biebel (2001). In one embodiment of the invention the media is as described in the Examples section herein after.

Typically, the CO will be added to the fermentation reaction in a gaseous state. However, methods of the invention are not limited to addition of the substrate in this state. For example, the carbon monoxide can be provided in a liquid. For example, a liquid may be saturated with a carbon monoxide containing gas and that liquid added to the bioreactor. This may be achieved using standard methodology. By way of example a microbubble dispersion generator (Hensirisak et. al. Scale-up of microbubble dispersion generator for aerobic fermentation; Applied Biochemistry and Biotechnology Volume 101, Number 3 / October, 2002) could be used for this purpose. Where a "gas stream" is referred to herein, the term also encompasses other forms of transporting the gaseous components of that stream such as the saturated liquid method described above.

### The Gaseous Substrate

The CO-containing substrate may contain any proportion of CO, such as at least about 20% to about 100% CO by volume, from 40% to 95% CO by volume, from 40% to 60% CO by volume, and from 45% to 55% CO by volume. In particular embodiments, the substrate comprises about 25%, or about 30%, or about 35%, or about 40%, or about 45%, or about 50% CO, or about 55% CO, or about 60% CO by volume. Substrates having lower concentrations of CO, such as 2%, may also be appropriate, particularly when H₂ and CO₂ are also present.

The presence of H₂ should not be detrimental to hydrocarbon product formation by fermentation. In particular embodiments, the presence of hydrogen results in an improved overall efficiency of alcohol production. For example, in particular embodiments, the substrate may comprise an approximate 2:1, or 1:1, or 1:2 ratio of H₂:CO. In other embodiments, the CO containing substrate comprises less than about 30% H₂, or less than 27% H₂, or less than 20 % H₂, or less than 10% H₂, or lower concentrations of H₂, for example, less than 5%, or less than 4%, or less than 3%, or less than 2%, or less than 1%, or is substantially hydrogen free. In still other embodiments, the CO containing substrate comprises greater than 50 % H2, or greater than 60% H₂, or greater than 70% H₂, or greater than 80% H₂, or greater than 90% H₂. The substrate may also contain some CO₂ for example, such as about 1% to about 80% CO₂ by volume, or 1% to about 30% CO₂ by volume.

### Fermentation conditions and microorganisms

Processes for the production of ethanol and other alcohols from gaseous substrates are known. Exemplary processes include those described for example in WO2007/117157, WO2008/115080, WO2009/022925, WO2009/064200, US 6,340,581, US 6,136,577, US 5,593,886, US 5,807,722 and US 5,821,111.

The fermentation should desirably be carried out under appropriate fermentation conditions for the production of desirable fermentation products to occur. Reaction conditions that should be considered include pressure, temperature, gas flow rate, liquid flow rate, media pH, media redox potential, agitation rate (if using a continuous stirred tank reactor), inoculum level, maximum gas substrate concentrations to ensure that CO in the liquid phase does not become limiting, and maximum product concentrations to avoid product inhibition.

In addition, it is often desirable to increase the CO concentration of a substrate stream (or CO partial pressure in a gaseous substrate) and thus increase the efficiency of fermentation reactions where CO is a substrate. Operating at increased pressures allows a significant increase in the rate of CO transfer from the gas phase to the liquid phase where it can be taken up by the micro-organism as a carbon source for the production of fermentation. This in turn means that the retention time (defined as the liquid volume in the bioreactor divided by the input gas flow rate) can be reduced when bioreactors are maintained at elevated pressure rather than atmospheric pressure. The optimum reaction conditions will depend partly on the particular micro-organism of the invention used. However, in general, it is preferred that the fermentation be performed at pressure higher than ambient pressure. Also, since a given CO-to-product conversion rate is in part a function of the substrate retention time, and achieving a desired retention time in turn dictates the required volume of a bioreactor, the use of pressurized systems can greatly reduce the volume of the bioreactor required, and consequently the capital cost of the fermentation equipment. According to examples given in US 5,593,886 reactor volume can be reduced in linear proportion to increases in reactor operating pressure, i.e. bioreactors operated at 10 atmospheres of pressure need only be one tenth the volume of those operated at 1 atmosphere of pressure.

By way of example, the benefits of conducting a gas-to-ethanol fermentation at elevated pressures has been described. For example, WO 02/08438 describes gas-to-ethanol fermentations performed under pressures of 30 psig (310000 Pa) and 75 psig (620000 Pa), giving ethanol productivities of 150 g/l/day and 369 g/l/day respectively. However, example fermentations performed using similar media and input gas compositions at atmospheric pressure were found to produce between 10 and 20 times less ethanol per litre per day.

It is also desirable that the rate of introduction of the CO-containing gaseous substrate is such as to ensure that the concentration of CO in the liquid phase does not become limiting. This is because a consequence of CO-limited conditions may be that one or more product is consumed by the culture.

The composition of gas streams used to feed a fermentation reaction can have a significant impact on the efficiency and/or costs of that reaction. For example, O₂ may reduce the efficiency of an anaerobic fermentation process. Processing of unwanted or unnecessary gases in stages of a fermentation process before or after fermentation can increase the burden on such stages (e.g. where the gas stream is compressed before entering a bioreactor, unnecessary energy may be used to compress gases that are not needed in the fermentation). Accordingly, it may be desirable to treat substrate streams, particularly substrate streams derived from industrial sources, to remove unwanted components and increase the concentration of desirable components.

In certain embodiments a culture of a microorganism defined herein is maintained in an aqueous culture medium. Preferably the aqueous culture medium is a minimal anaerobic microbial growth medium. Suitable media are known in the art and described for example in US 5,173,429 and 5,593,886 and WO 02/08438, and as described in the Examples section herein after.

In a particular embodiment, the microorganism is selected from the group of carboxydotrophic acetogenic bacteria comprising Clostridium autoethanogenum, Clostridium ljungdahlii, Clostridium ragsdalei, Clostridium carboxidivorans, Clostridium drakei, Clostridium scatologenes, Clostridium aceticum, Clostridium formicoaceticum, Clostridium magnum, Butyribacterium methylotrophicum, Acetobacterium woodii, Alkalibaculum bacchii, Blautia producta, Eubacterium limosum, Moorella thermoacetica, Moorella thermautotrophica, Sporomusa ovata, Sporomusa silvacetica, Sporomusa sphaeroides, Oxobacter pfennigii, and Thermoanaerobacter kiuvi.

In one particular embodiment, the parental microorganism is selected from the cluster of ethanologenic, acetogenic Clostridia comprising the species C. autoethanogenum, C. ljungdahlii, and C. ragsdalei and related isolates. These include but are not limited to strains C. autoethanogenum JAI-1T (DSM10061) [Abrini J, Naveau H, Nyns E-J: Clostridium autoethanogenum, sp. nov., an anaerobic bacterium that produces ethanol from carbon monoxide. Arch Microbiol 1994, 4: 345-351], C. autoethanogenum LBS1560 (DSM19630) [Simpson SD, Forster RL, Tran PT, Rowe MJ, Warner IL: Novel bacteria and methods thereof. International patent application WO/2009/064200], C. autoethanogenum LBS1561 (DSM23693), C. ljungdahlii PETCT (DSM13528 = ATCC 55383) [Tanner RS, Miller LM, Yang D: Clostridium ljungdahlii sp. nov., an Acetogenic Species in Clostridial rRNA Homology Group I. Int J Syst Bacteriol 1993, 43: 232-236], C. ljungdahlii ERI-2 (ATCC 55380) [Gaddy JL: Clostridium stain which produces acetic acid from waste gases. US 5,593,886], C. ljungdahlii C-01 (ATCC 55988) [Gaddy JL, Clausen EC, Ko C-W: Microbial process for the preparation of acetic acid as well as solvent for its extraction from the fermentation broth. US 6,368,819], C. ljungdahlii O-52 (ATCC 55989) [Gaddy JL, Clausen EC, Ko C-W: Microbial process for the preparation of acetic acid as well as solvent for its extraction from the fermentation broth. US 6,368,819], C. ragsdalei P11T (ATCC BAA-622) [Huhnke RL, Lewis RS, Tanner RS: Isolation and Characterization of novel Clostridial Species. International patent application WO 2008/028055], related isolates such as "C. coskatii" [Zahn et al - Novel ethanologenic species Clostridium coskatii (US 20110229947)] and "Clostridium sp." (Tyurin et al., 2012, J. Biotech Res. 4: 1-12), or mutated strains such as C. ljungdahlii OTA-1 (Tirado-Acevedo O. Production of Bioethanol from Synthesis Gas Using Clostridium ljungdahlii. PhD thesis, North Carolina State University, 2010). These strains form a subcluster within the Clostridial rRNA cluster I , and their 16S rRNA gene is more than 99% identical with a similar low GC content of around 30%. However, DNA-DNA reassociation and DNA fingerprinting experiments showed that these strains belong to distinct species [Huhnke RL, Lewis RS, Tanner RS: Isolation and Characterization of novel Clostridial Species. International patent application WO 2008/028055]. All species of this cluster have a similar morphology and size (logarithmic growing cells are between 0.5-0.7 x 3-5 µm), are mesophilic (optimal growth temperature between 30-37 °C) and strictly anaerobe [Tanner RS, Miller LM, Yang D: Clostridium ljungdahlii sp. nov., an Acetogenic Species in Clostridial rRNA Homology Group I. Int J Syst Bacteriol 1993, 43: 232-236; Abrini J, Naveau H, Nyns E-J: Clostridium autoethanogenum, sp. nov., an anaerobic bacterium that produces ethanol from carbon monoxide. Arch Microbiol 1994, 4: 345-351; Huhnke RL, Lewis RS, Tanner RS: Isolation and Characterization of novel Clostridial Species. International patent application WO 2008/028055]. Moreover, they all share the same major phylogenetic traits, such as same pH range (pH 4-7.5, with an optimal initial pH of 5.5-6), strong autotrophic growth on CO containing gases with similar growth rates, and a similar metabolic profile with ethanol and acetic acid as main fermentation end product, and small amounts of 2,3-butanediol and lactic acid formed under certain conditions. [Tanner RS, Miller LM, Yang D: Clostridium ljungdahlii sp. nov., an Acetogenic Species in Clostridial rRNA Homology Group I. Int J Syst Bacteriol 1993, 43: 232-236; Abrini J, Naveau H, Nyns E-J: Clostridium autoethanogenum, sp. nov., an anaerobic bacterium that produces ethanol from carbon monoxide. Arch Microbiol 1994, 4: 345-351; Huhnke RL, Lewis RS, Tanner RS: Isolation and Characterization of novel Clostridial Species. International patent application WO 2008/028055]. Indole production was observed with all three species as well. However, the species differentiate in substrate utilization of various sugars (e.g. rhamnose, arabinose), acids (e.g. gluconate, citrate), amino acids (e.g. arginine, histidine), or other substrates (e.g. betaine, butanol). Moreover some of the species were found to be auxotroph to certain vitamins (e.g. thiamine, biotin) while others were not.

In one embodiment the parental microorganism is *Clostridium autoethanogenum* or *Clostridium ljungdahlii.* In one particular embodiment, the microorganism is *Clostridium autoethanogenum* DSM23693. In another particular embodiment, the microorganism is *Clostridium ljungdahlii* DSM13528 (or ATCC55383).

The fermentation may be carried out in any suitable bioreactor configured for gas/liquid contact wherein the substrate can be contacted with one or more microorganisms, such as a continuous stirred tank reactor (CSTR), an immobilised cell reactor, a gas-lift reactor, a bubble column reactor (BCR), a membrane reactor, such as a Hollow Fibre Membrane Bioreactor (HFMBR) or a trickle bed reactor (TBR), monolith bioreactor or loop reactors. Also, in some embodiments of the invention, the bioreactor may comprise a first, growth reactor in which the micro-organisms are cultured, and a second, fermentation reactor, to which fermentation broth from the growth reactor is fed and in which most of the fermentation product (e.g. ethanol and acetate) is produced.

According to various embodiments of the invention, the carbon source for the fermentation reaction is syngas derived from gasification. The syngas substrate will typically contain a major proportion of CO, such as at least about 15% to about 75% CO by volume, from 20% to 70% CO by volume, from 20% to 65% CO by volume, from 20% to 60% CO by volume, and from 20% to 55% CO by volume. In particular embodiments, the substrate comprises about 25%, or about 30%, or about 35%, or about 40%, or about 45%, or about 50% CO, or about 55% CO, or about 60% CO by volume. Substrates having lower concentrations of CO, such as 6%, may also be appropriate, particularly when H₂ and CO₂ are also present. In particular embodiments, the presence of hydrogen results in an improved overall efficiency of alcohol production. The gaseous substrate may also contain some CO₂ for example, such as about 1% to about 80% CO₂ by volume, or 1% to about 30% CO₂ by volume.

In accordance with particular embodiments of the invention, the CO content and/or the H₂ content of the reformed substrate stream can be enriched prior to passing the stream to the bioreactor. For example, hydrogen can be enriched using technologies well known in the art, such as pressure swing adsorption, cryogenic separation and membrane separation. Similarly, CO can be enriched using technologies well known in the art, such as copper-ammonium scrubbing, cryogenic separation, COSORB™ technology (absorption into cuprous aluminium dichloride in toluene), vacuum swing adsorption and membrane separation. Other methods used in gas separation and enrichment are detailed in PCT/NZ2008/000275 (WO 2009/058028).

Typically, the carbon monoxide will be added to the fermentation reaction in a gaseous state. However, the methods of the invention are not limited to addition of the substrate in this state. For example, the carbon monoxide can be provided in a liquid. For example, a liquid may be saturated with a carbon monoxide containing gas and that liquid added to the bioreactor. This may be achieved using standard methodology. By way of example a microbubble dispersion generator (Hensirisak et. al. Scale-up of microbubble dispersion generator for aerobic fermentation; Applied Biochemistry and Biotechnology Volume 101, Number 3 / October, 2002) could be used for this purpose.

It will be appreciated that for growth of the bacteria and CO-to-alcohol fermentation to occur, in addition to the CO-containing substrate gas, a suitable liquid nutrient medium will need to be fed to the bioreactor. A nutrient medium will contain vitamins and minerals sufficient to permit growth of the micro-organism used. Anaerobic media suitable for the fermentation of ethanol using CO as the sole carbon source are known in the art. For example, suitable media are described in US 5,173,429 and 5,593,886 and WO 02/08438, WO2007/117157, WO2008/115080, WO2009/022925, WO2009/058028, WO2009/064200, WO2009/064201, WO2009/113878 and WO2009/151342 referred to above. Also described is a novel media which has increased efficacy in supporting growth of the micro-organisms and/or alcohol production in the fermentation process. This media will be described in more detail hereinafter.

The fermentation should desirably be carried out under appropriate conditions for the desired fermentation to occur (e.g. CO-to-ethanol). Reaction conditions that should be considered include pressure, temperature, gas flow rate, liquid flow rate, media pH, media redox potential, agitation rate (if using a continuous stirred tank reactor), inoculum level, maximum gas substrate concentrations to ensure that CO in the liquid phase does not become limiting, and maximum product concentrations to avoid product inhibition. Suitable conditions are described in WO02/08438, WO2007/117157, WO2008/115080, WO2009/022925, WO2009/058028, WO2009/064200, WO2009/064201, WO2009/113878 and WO2009/151342.

The optimum reaction conditions will depend partly on the particular micro-organism used. However, in general, it is preferred that the fermentation be performed at pressure higher than ambient pressure. Operating at increased pressures allows a significant increase in the rate of CO transfer from the gas phase to the liquid phase where it can be taken up by the micro-organism as a carbon source for the production of ethanol. This in turn means that the retention time (defined as the liquid volume in the bioreactor divided by the input gas flow rate) can be reduced when bioreactors are maintained at elevated pressure rather than atmospheric pressure.

The benefits of conducting a gas-to-ethanol fermentation at elevated pressures have also been described elsewhere. For example, WO 02/08438 describes gas-to-ethanol fermentations performed under pressures of 30 psig (310000 Pa) and 75 psig (620000 Pa), giving ethanol productivities of 150 g/l/day and 369 g/l/day respectively. However, example fermentations performed using similar media and input gas compositions at atmospheric pressure were found to produce between 10 and 20 times less ethanol per litre per day.

It is also desirable that the rate of introduction of the CO and H₂ containing gaseous substrate is such as to ensure that the concentration of CO in the liquid phase does not become limiting. This is because a consequence of CO-limited conditions may be that the ethanol product is consumed by the culture.

Fermentation products such as ethanol, or mixed streams containing more than one fermentation products, may be recovered from the fermentation broth by methods known in the art, such as fractional distillation or evaporation, pervaporation, gas stripping and extractive fermentation, including for example, liquid-liquid extraction. Products may also diffuse or secrete into media, from which they can extracted by phase separation.

In certain preferred embodiments of the invention, one or more products are recovered from the fermentation broth by continuously removing a portion of the broth from the bioreactor, separating microbial cells from the broth (conveniently by filtration), and recovering one or more products from the broth. Alcohols may conveniently be recovered for example by distillation. Acetone may be recovered for example by distillation. Any acids produced may be recovered for example by adsorption on activated charcoal. The separated microbial cells are preferably returned to the fermentation bioreactor. The cell free permeate remaining after any alcohol(s) and acid(s) have been removed is also preferably returned to the fermentation bioreactor. Additional nutrients (such as B vitamins) may be added to the cell free permeate to replenish the nutrient medium before it is returned to the bioreactor.

Also, if the pH of the broth was adjusted as described above to enhance adsorption of acetic acid to the activated charcoal, the pH should be re-adjusted to a similar pH to that of the broth in the fermentation bioreactor, before being returned to the bioreactor

### Product recovery

Then products of the fermentation reaction can be recovered using known methods. Exemplary methods include those described in WO2007/117157, WO2008/115080, WO2009/022925, US 6,340,581, US 6,136,577, US 5,593,886, US 5,807,722 and US 5,821,111. However, briefly and by way of example only ethanol may be recovered from the fermentation broth by methods such as fractional distillation or evaporation, and extractive fermentation.

Distillation of ethanol from a fermentation broth yields an azeotropic mixture of ethanol and water (i.e., 95% ethanol and 5% water). Anhydrous ethanol can subsequently be obtained through the use of molecular sieve ethanol dehydration technology, which is also well known in the art.

Extractive fermentation procedures involve the use of a water-miscible solvent that presents a low toxicity risk to the fermentation organism, to recover the ethanol from the dilute fermentation broth. For example, oleyl alcohol is a solvent that may be used in this type of extraction process. Oleyl alcohol is continuously introduced into a fermenter, whereupon this solvent rises forming a layer at the top of the fermenter which is continuously extracted and fed through a centrifuge. Water and cells are then readily separated from the oleyl alcohol and returned to the fermenter while the ethanol-laden solvent is fed into a flash vaporization unit. Most of the ethanol is vaporized and condensed while the oleyl alcohol is non volatile and is recovered for re-use in the fermentation.

Acetate, which is produced as by-product in the fermentation reaction, may also be recovered from the fermentation broth using methods known in the art. For example, an adsorption system involving an activated charcoal filter may be used. In this case, it is preferred that microbial cells are first removed from the fermentation broth using a suitable separation unit. Numerous filtration-based methods of generating a cell free fermentation broth for product recovery are known in the art. The cell free ethanol - and acetate - containing permeate is then passed through a column containing activated charcoal to adsorb the acetate. Acetate in the acid form (acetic acid) rather than the salt (acetate) form is more readily adsorbed by activated charcoal. It is therefore preferred that the pH of the fermentation broth is reduced to less than about 3 before it is passed through the activated charcoal column, to convert the majority of the acetate to the acetic acid form.

Acetic acid adsorbed to the activated charcoal may be recovered by elution using methods known in the art. For example, ethanol may be used to elute the bound acetate. In certain embodiments, ethanol produced by the fermentation process itself may be used to elute the acetate. Because the boiling point of ethanol is 78.8 °C and that of acetic acid is 107 °C, ethanol and acetate can readily be separated from each other using a volatility-based method such as distillation.

Other methods for recovering acetate from a fermentation broth are also known in the art and may be used in the processes of the present invention. For example, US 6,368,819 and 6,753,170 describe a solvent and cosolvent system that can be used for extraction of acetic acid from fermentation broths. As with the example of the oleyl alcohol-based system described for the extractive fermentation of ethanol, the systems described in US 6,368,819 and 6,753,170 describe a water immiscible solvent/co-solvent that can be mixed with the fermentation broth in either the presence or absence of the fermented micro-organisms in order to extract the acetic acid product. The solvent/co-solvent containing the acetic acid product is then separated from the broth by distillation. A second distillation step may then be used to purify the acetic acid from the solvent/co-solvent system.

The products of the fermentation reaction (for example ethanol and acetate) may be recovered from the fermentation broth by continuously removing a portion of the broth from the fermentation bioreactor, separating microbial cells from the broth (conveniently by filtration), and recovering one or more product from the broth simultaneously or sequentially. In the case of ethanol it may be conveniently recovered by distillation, and acetate may be recovered by adsorption on activated charcoal, using the methods described above. The separated microbial cells are preferably returned to the fermentation bioreactor. The cell free permeate remaining after the ethanol and acetate have been removed is also preferably returned to the fermentation bioreactor. Additional nutrients (such as B vitamins) may be added to the cell free permeate to replenish the nutrient medium before it is returned to the bioreactor. Also, if the pH of the broth was adjusted as described above to enhance adsorption of acetic acid to the activated charcoal, the pH should be re-adjusted to a similar pH to that of the broth in the fermentation bioreactor, before being returned to the bioreactor.

### General

While the invention is broadly described with reference to any type of stream that may be moved through or around the system(s) by any known transfer means, in certain embodiments, the biogas and reformed and/or blended substrate streams are gaseous. Those skilled in the art will appreciate that particular stages may be coupled by suitable conduit means or the like, configurable to receive or pass streams throughout a system. A pump or compressor may be provided to facilitate delivery of the streams to particular stages. Furthermore, a compressor can be used to increase the pressure of gas provided to one or more stages, for example the bioreactor. As discussed hereinabove, the pressure of gases within a bioreactor can affect the efficiency of the fermentation reaction performed therein. Thus, the pressure can be adjusted to improve the efficiency of the fermentation. Suitable pressures for common reactions are known in the art.

In addition, the processes of the invention may optionally include means for regulating and/or controlling other parameters to improve overall efficiency of the process. For example particular embodiments may include determining means to monitor the composition of substrate and/or exhaust stream(s). In addition, particular embodiments may include a means for controlling the delivery of substrate stream(s) to particular stages or elements within a particular system if the determining means determines the stream has a composition suitable for a particular stage. For example, in instances where a gaseous substrate stream contains low levels of CO or high levels of O₂ that may be detrimental to a fermentation reaction, the substrate stream may be diverted away from the bioreactor. The system may include means for monitoring and controlling the destination of a substrate stream and/or the flow rate, such that a stream with a desired or suitable composition can be delivered to a particular stage.

In addition, it may be necessary to heat or cool particular system components or substrate stream(s) prior to or during one or more stages in the process. In such instances, known heating or cooling means may be used.

The systems are described in the accompanying Figures.

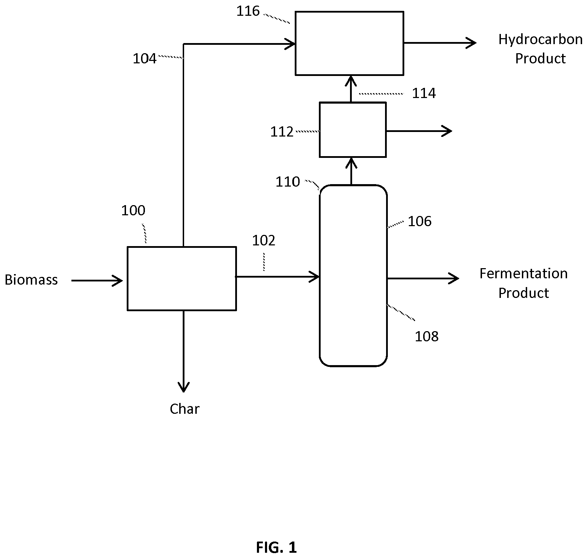

Figure 1 shows a system and method for the production of one or more products, the system including;
**a.** a pyrolysis zone **100** wherein a biomass feedstock is reacted under pyrolysis conditions to produce a gaseous substrate and at least one pyrolysis product, selected from the group consisiting of pyrolysis and char.
**b.** a bioreactor **106** adapted to receive the gaseous substrate from the pyrolysis zone via a conduit **102**. The bioreactor **106** contains a culture of at least one carboxydotrophic acetogenic microorganism in a liquid nutrient medium broth. The bioreactor is opereated under fermentation conditions to produce at least one fermentation product and an exit gas stream comprising H2. The at least one fermentation product is removed from the reactor via a product conduit **108**.
**c.** the exit stream is passed from the bioreactor via a gas conduit to a gas separation zone **112**, where a hydrogen portion of the exit gas stream is separated and passed via a conduit **114** to a hydrogenation zone **116**.
**d.** The hydrogenation zone **116** is adapted to receive a hydrogen stream from the gas separation zone and the pyrolysis oil from the pyrolysis zone via a conduit **104**. The pyrolysis oil and the hydrogen are reacted under hydrogenation conditions to produce a hydrocarbon product having between 6 and 20 carbons.

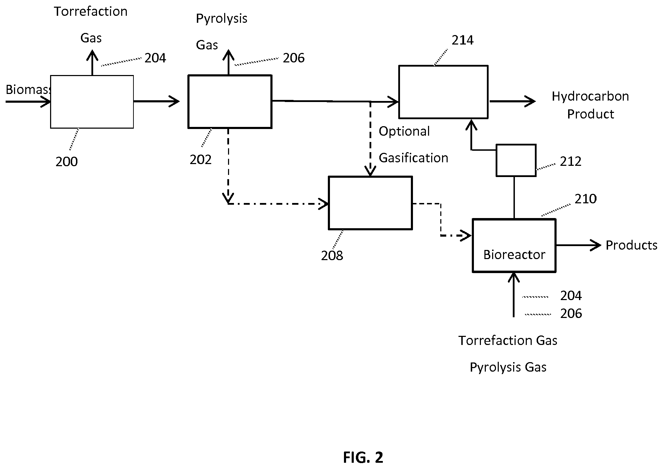

Figure 2 shows the production of one or more products by fermentation of a gasesous substrate produced by liquefaction of biomass. The biomass feedstock is passed to a Torrefaction zone **200** wherein the torrefaction zone operates under conditions to produce a torrefied biomass and a torrefaction gas stream **204**. The torrefied biomass is passed to a pyrolysis zone **202**. The torrified biomass is reacted under pyrolysis conditions to produce a pyrolysis gas stream **206**, pyrolysis oil and char. At least a portion of the torrefecation gas stream **204** and the pyrolysis gas stream **206** are passed to a bioreactor **210**.

At least a portion of the pyrolysis oil and/or char can optionally be passed to a gasification zone **208** where they are gasified to produce a gasification substrate comprising CO. The gasification substrate can be passed to the bioreactor **210**. The bioreactor **210** contains a culture of at least one carboxydotrophic acetogenic microorganism in a liquid nutrient medium broth. The bioreactor is opereated under fermentation conditions to produce at least one fermentation product and an exit gas stream comprising H2. The exit stream is passed to a gas separation zone **212** where hydrogen is separated from the gas stream to produce an enriched hydrogen stream. The enriched hydrogen stream is passed to a hydrogenation zone **214**. The hydrogenation zone **214** is adapted to receive the pyrolysis oil from the pyrolysis zone **206** and the enriched hydrogen stream. The pyrolysis oil and hydrogen stream are reacted under hydrogenation coniditons to produce a hydrocarbon having between 6 and 20 carbons.

Throughout this specification and any claims which follow, unless the context requires otherwise, the words "comprise", "comprising" and the like, are to be construed in an inclusive sense as opposed to an exclusive sense, that is to say, in the sense of "including, but not limited to".

## Claims

1. A method for producing at least one product from a gaseous substrate, the method comprising:
(I)
(a) converting at least a portion of a biomass feedstock to a gaseous substrate comprising CO by pyrolysis or torrefaction, the process being carried out in a liquefaction zone;
(b) passing at least a portion of the gaseous substrate to a bioreactor comprising a culture of at least one carboxydotrophic acetogenic microorganism, and anaerobically fermenting at least a portion of the gaseous substrate to produce at least one fermentation product and a waste stream comprising a second biomass;
(c) separating at least a portion of the second biomass from the waste stream; and
(d) Passing a portion of the second biomass to the liquefaction zone; or
(II)
(a) passing a biomass feedstock to a pyrolysis zone, operated at conditions to produce a gaseous substrate comprising CO, CO₂ and H₂, and at least one pyrolysis product selected from oil and char;
(b) passing at least a portion of the gaseous substrate to a bioreactor comprising a culture of at least one carboxydotrophic acetogenic microorganism, and anaerobically fermenting at least a portion of the gaseous substrate to produce at least one fermentation product, a waste stream comprising a second biomass and an exit gas stream comprising hydrogen;
(c) separating at least a portion of the second biomass from the waste stream; and
(d) passing a portion of the second biomass to the pyrolysis zone; or
(III)
(a) passing a biomass feedstock to a torrefaction zone to produce a torrefied biomass;
(b) passing the torrefied biomass to a pyrolysis zone to produce a gaseous substrate comprising CO, pyrolysis oil and char;
(c) passing at least a portion of the gaseous substrate to a bioreactor comprising a culture of at least one carboxydotrophic acetogenic microorganism selected from the group consisting of *Clostridium autoethanogenum*, *Clostridium* ljungdahlii *Clostridium ragsdalei* and *Clostridium coskatii*, and anaerobically fermenting at least a portion of the gaseous substrate to produce at least one fermentation product, a waste stream comprising a second biomass and an exit gas stream comprising hydrogen;
(d) separating at least a portion of the second biomass from the waste stream;
(e) passing the exit gas stream to a separation zone operated at conditions to provide a hydrogen rich stream;
(f) passing a portion of the second biomass to the torrefaction zone; and
(g) passing the pyrolysis oil and the hydrogen rich stream to a hydrogenation zone operated at conditions to produce a hydrogenated product having from 6 to 20 carbons.

2. The method of claim 1, which is Alternative (I).

3. The method of claim 2, wherein the gaseous substrate further comprises CO₂ and H₂.

4. The method of claim 3 wherein the pyrolysis or torrefaction produces at least one non-gaseous product that is gasified to produce a syngas stream comprising CO.

5. The method of claim 4 wherein at least a portion of the syngas stream is passed to the bioreactor.

6. The method of claim 2 wherein the at least one fermentation product is selected from ethanol, acetic acid and 2,3-butanediol.

7. The method of claim 1, which is Alternative (II).

8. The method of claim 7 wherein the exit gas stream is passed to a separation zone operated at conditions to provide a hydrogen rich stream.

9. The method of claim 8 where the pyrolysis product is pyrolysis oil and the hydrogen rich stream and the pyrolysis oil are passed to a hydrogenation zone operated at conditions to produce a hydrogenated product.

10. The method of claim 9 wherein the hydrogenated product is a hydrocarbon having from 6 to 20 carbons.

11. The method of claim 7 further comprising passing the gaseous substrate from the pyrolysis zone to a separation zone operated at conditions to provide a CO₂ rich stream and an enriched CO and H₂ gaseous substrate and passing the enriched gaseous substrate to the bioreactor.

12. The method of claim 11 where the pyrolysis product is char and the char and the CO₂ rich stream are passed to a reaction zone to produce a second substrate stream comprising CO, and passing the second substrate stream to the bioreactor.

13. The method of claim 7, further comprising prior to passing the biomass feedstock to the pyrolysis zone, the biomass feedstock is first passed to a torrefaction zone.

14. The method of claim 1, which is Alternative (III).

15. The method of claim 14 wherein the torrefaction process produces a gaseous by-product stream comprising CO, and at least a portion of the gaseous by-product stream is passed to the bioreactor; or
wherein the char is passed to a gasification zone and gasified to produce a second gaseous substrate comprising CO, and passing the second gaseous substrate to the bioreactor.

## Patentansprüche

1. Verfahren zum Herstellen von mindestens einem Produkt aus einem gasförmigen Substrat, wobei das Verfahren Folgendes umfasst:
(I)
(a) Umwandeln von mindestens einem Teil eines Biomasse-Rohmaterials in ein gasförmiges Substrat, das CO umfasst, durch Pyrolyse oder Torrefizierung, wobei der Prozess in einem Verflüssigungsbereich ausgeführt wird;
(b) Weitergeben von mindestens einem Teil des gasförmigen Substrats in einen Bioreaktor, umfassend eine Kultur von mindestens einem carboxydotrophen acetogenen Mikroorganismus, und anaerobes Fermentieren von mindestens einem Teil des gasförmigen Substrats, um mindestens ein Fermentationsprodukt und einen Abfallstrom, der eine zweite Biomasse umfasst, herzustellen;
(c) Trennen von mindestens einem Teil der zweiten Biomasse vom Abfallstrom; und
(d) Weitergeben eines Teils der zweiten Biomasse in den Verflüssigungsbereich; oder
(II)
(a) Weitergeben eines Biomasse-Rohmaterials in einen Pyrolysebereich, durchgeführt bei Bedingungen, um ein gasförmiges Substrat, das CO, CO₂ und H₂ umfasst, und mindestens ein Pyrolyseprodukt, ausgewählt aus Öl und Kohle, herzustellen;
(b) Weitergeben von mindestens einem Teil des gasförmigen Substrats in einen Bioreaktor, umfassend eine Kultur von mindestens einem carboxydotrophen acetogenen Mikroorganismus, und anaerobes Fermentieren von mindestens einem Teil des gasförmigen Substrats, um mindestens ein Fermentationsprodukt, einen Abfallstrom, der eine zweite Biomasse umfasst, und einen Austrittsgasstrom, der Wasserstoff umfasst, herzustellen;
(c) Trennen von mindestens einem Teil der zweiten Biomasse vom Abfallstrom; und
(d) Weitergeben eines Teils der zweiten Biomasse in den Pyrolysebereich; oder
(III)
(a) Weitergeben eines Biomasse-Rohmaterials in einen Torrefizierungsbereich, um eine torrefizierte Biomasse herzustellen;
(b) Weitergeben der torrefizierten Biomasse in einen Pyrolysebereich, um ein gasförmiges Substrat herzustellen, das CO, Pyrolyseöl und Kohle umfasst;
(c) Weitergeben von mindestens einem Teil des gasförmigen Substrats in einen Bioreaktor, umfassend eine Kultur von mindestens einem carboxydotrophen acetogenen Mikroorganismus, ausgewählt aus der Gruppe bestehend aus *Clostridium autoethanogenum*, *Clostridium ljungdahlii*, *Clostridium ragsdalei* und *Clostridium coskatii*, und anaerobes Fermentieren von mindestens einem Teil des gasförmigen Substrats, um mindestens ein Fermentationsprodukt, einen Abfallstrom, der eine zweite Biomasse umfasst, und einen Austrittsgasstrom, der Wasserstoff umfasst, herzustellen;
(d) Trennen von mindestens einem Teil der zweiten Biomasse vom Abfallstrom;
(e) Weitergeben des Austrittsgasstroms in einen Trennbereich, durchgeführt bei Bedingungen, um einen wasserstoffreichen Strom bereitzustellen;
(f) Weitergeben eines Teils der zweiten Biomasse in den Torrefizierungsbereich; und
(g) Weitergeben des Pyrolyseöls und des wasserstoffreichen Stroms in einen Hydrierbereich, durchgeführt bei Bedingungen, um ein hydriertes Produkt herzustellen, das 6 bis 20 Kohlenstoffatome hat.

2. Verfahren nach Anspruch 1, das Alternative (I) ist.

3. Verfahren nach Anspruch 2, wobei das gasförmige Substrat ferner CO₂ und H2 umfasst.

4. Verfahren nach Anspruch 3, wobei die Pyrolyse oder Torrefizierung mindestens ein nicht gasförmiges Produkt herstellt, das vergast wird, um einen Synthesegasstrom herzustellen, der CO umfasst.

5. Verfahren nach Anspruch 4, wobei mindestens ein Teil des Synthesegasstroms in den Bioreaktor weitergegeben wird.

6. Verfahren nach Anspruch 2, wobei das mindestens eine Fermentationsprodukt aus Ethanol, Essigsäure und 2,3-Butandiol ausgewählt ist.

7. Verfahren nach Anspruch 1, das Alternative (II) ist.

8. Verfahren nach Anspruch 7, wobei der Austrittsgasstrom in einen Trennbereich weitergegeben wird, durchgeführt bei Bedingungen, um einen wasserstoffreichen Strom bereitzustellen.

9. Verfahren nach Anspruch 8, wobei das Pyrolyseprodukt Pyrolyseöl ist und der wasserstoffreiche Strom und das Pyrolyseöl in einen Hydrierbereich weitergegeben werden, durchgeführt bei Bedingungen, um ein hydriertes Produkt herzustellen.

10. Verfahren nach Anspruch 9, wobei das hydrierte Produkt ein Kohlenwasserstoff ist, der 6 bis 20 Kohlenstoffatome hat.

11. Verfahren nach Anspruch 7, das ferner ein Weitergeben des gasförmigen Substrats vom Pyrolysebereich in einen Trennbereich, durchgeführt bei Bedingungen, um einen CO₂-reichen Strom und ein angereichertes gasförmiges CO- und H₂-Substrat herzustellen, und ein Weitergeben des angereicherten gasförmigen Substrats in den Bioreaktor umfasst.

12. Verfahren nach Anspruch 11, wobei das Pyrolyseprodukt Kohle ist und die Kohle und der CO₂-reiche Strom in einen Reaktionsbereich weitergegeben werden, um einen zweiten Substratstrom herzustellen, der CO umfasst, und Weitergeben des zweiten Substratstroms in den Bioreaktor.

13. Verfahren nach Anspruch 7, ferner umfassend vor dem Weitergeben des Biomasse-Rohmaterials in den Pyrolysebereich, dass das Biomasse-Rohmaterial zuerst in einen Torrefizierungsbereich weitergegeben wird.

14. Verfahren nach Anspruch 1, das Alternative (III) ist.

15. Verfahren nach Anspruch 14, wobei der Torrefizierungsprozess einen gasförmigen Nebenproduktstrom herstellt, der CO umfasst, und mindestens ein Teil des gasförmigen Nebenproduktstroms in den Bioreaktor weitergegeben wird; oder
wobei die Kohle in einen Vergasungsbereich weitergegeben wird und vergast wird, um ein zweites gasförmiges Substrat herzustellen, das CO umfasst, und Weitergeben des zweiten gasförmigen Substrats in den Bioreaktor.

## Revendications

1. Procédé de production d'au moins un produit à partir d'un substrat gazeux, le procédé comprenant :
(I)
(a) la conversion d'au moins une partie d'une charge d'alimentation de biomasse en un substrat gazeux comprenant CO par pyrolyse ou torréfaction, le procédé étant effectué dans une zone de liquéfaction ;
(b) le passage d'au moins une partie du substrat gazeux vers un bioréacteur comprenant une culture d'au moins un microorganisme acétogénique carboxydotrophique, et la fermentation anaérobie d'au moins une partie du substrat gazeux pour produire au moins un produit de fermentation et un flux de déchets comprenant une deuxième biomasse ;
(c) la séparation d'au moins une partie de la deuxième biomasse du flux de déchets ; et
(d) le passage d'une partie de la deuxième biomasse vers la zone de liquéfaction ; ou
(II)
(a) le passage d'une charge d'alimentation de biomasse vers une zone de pyrolyse, mis en oeuvre dans des conditions pour produire un substrat gazeux comprenant CO, CO₂ et H₂, et au moins un produit de pyrolyse choisi parmi l'huile et le charbon ;
(b) le passage d'au moins une partie du substrat gazeux vers un bioréacteur comprenant une culture d'au moins un microorganisme acétogénique carboxydotrophique, et la fermentation anaérobie d'au moins une partie du substrat gazeux pour produire au moins un produit de fermentation, un flux de déchets comprenant une deuxième biomasse et un flux de gaz de sortie comprenant l'hydrogène ;
(c) la séparation d'au moins une partie de la deuxième biomasse du flux de déchets ; et
(d) le passage d'une partie de la deuxième biomasse vers la zone de pyrolyse ; ou
(III)
(a) le passage d'une charge d'alimentation de biomasse vers une zone de torréfaction pour produire une biomasse torréfiée ;
(b) le passage de la biomasse torréfiée vers une zone de pyrolyse pour produire un substrat gazeux comprenant CO, l'huile et le charbon de pyrolyse ;
(b) le passage d'au moins une partie du substrat gazeux vers un bioréacteur comprenant une culture d'au moins un microorganisme acétogénique carboxydotrophique choisi dans le groupe constitué par *Clostridium autoethanogenum*, *Clostridium ljungdahlii*, *Clostridium ragsdalei* et *Clostridium coskatii*, et la fermentation anaérobie d'au moins une partie du substrat gazeux pour produire au moins un produit de fermentation, un flux de déchets comprenant une deuxième biomasse et un flux de gaz de sortie comprenant l'hydrogène ;
(d) la séparation d'au moins une partie de la deuxième biomasse du flux de déchets ;
(e) le passage du flux de gaz de sortie vers une zone de séparation mis en oeuvre dans des conditions pour fournir un flux riche en hydrogène ;
(f) le passage d'une partie de la deuxième biomasse vers la zone de torréfaction ; et
(g) le passage de l'huile de pyrolyse et du flux riche en hydrogène vers une zone d'hydrogénation mis en oeuvre dans des conditions pour produire un produit hydrogéné comportant de 6 à 20 atomes de carbone.

2. Procédé selon la revendication 1, qui est l'Alternative (I).

3. Procédé selon la revendication 2, dans lequel le substrat gazeux comprend en outre CO₂ et H₂.

4. Procédé selon la revendication 3, dans lequel la pyrolyse ou torréfaction produit au moins un produit non gazeux qui est gazéifié pour produire un flux de gaz de synthèse comprenant CO.

5. Procédé selon la revendication 4, dans lequel au moins une partie du flux de gaz de synthèse est transmise au bioréacteur.

6. Procédé selon la revendication 2 dans lequel l'au moins un produit de fermentation est choisi parmi l'éthanol, l'acide acétique et 2,3-butanediol.

7. Procédé selon la revendication 1, qui est l'Alternative (II).

8. Procédé selon la revendication 7, dans lequel le flux de gaz de sortie est transmis à une zone de séparation mise en oeuvre dans des conditions pour fournir un flux riche en hydrogène.

9. Procédé selon la revendication 8, où le produit de pyrolyse est l'huile de pyrolyse et le flux riche en hydrogène et l'huile de pyrolyse sont transmis à une zone d'hydrogénation mise en oeuvre dans des conditions pour produire un produit hydrogéné.

10. Procédé selon la revendication 9, dans lequel le produit hydrogéné est un hydrocarbure comportant de 6 à 20 atomes de carbone.

11. Procédé selon la revendication 7 comprenant en outre le passage du substrat gazeux de la zone de pyrolyse vers une zone de séparation mise en oeuvre dans des conditions pour fournir un flux riche en CO₂ et un substrat gazeux enrichi en CO et H₂ et le passage du substrat gazeux enrichi vers le bioréacteur.

12. Procédé selon la revendication 11 où le produit de pyrolyse est le charbon et le charbon et le flux riche en CO₂ sont transmis à une zone de réaction pour produire un deuxième flux de substrat comprenant CO, et le passage du deuxième flux de substrat vers le bioréacteur.

13. Procédé selon la revendication 7, comprenant en outre avant le passage de la charge d'alimentation de biomasse vers la zone de pyrolyse, la charge d'alimentation de biomasse est d'abord transmise à une zone de torréfaction.

14. Procédé selon la revendication 1, qui est l'Alternative (III).

15. Procédé selon la revendication 14, dans lequel le processus de torréfaction produit un flux de sous-produits gazeux comprenant CO, et au moins une partie du flux de sous-produits gazeux est transmise au bioréacteur ; ou
dans lequel le charbon est transmis à une zone de gazéification et gazéifié pour produire un deuxième substrat gazeux comprenant CO, et le passage du deuxième substrat gazeux vers le bioréacteur.
